# EUROPÄISCHE PATENTSCHRIFT

(11) **EP 1 341 758 B1**
(45) Veröffentlichungstag und Bekanntmachung des Hinweises auf die Patenterteilung: **28.06.2006**
(21) Anmeldenummer: 01989546.5
(22) Anmeldetag: 30.11.2001
(51) Int. Cl.: C07D 207/44, A61K 31/4015, A61P 31/04, A61P 31/10

(54) **CONIOSETIN UND DERIVATE DAVON, VERFAHREN ZUR HERSTELLUNG UND VERWENDUNG DERSELBEN**
CONIOSETIN AND DERIVATIVES THEREOF, METHOD FOR PRODUCING THE SAME AND USE THEREOF
CONIOSETINE ET SES DERIVES, LEUR PROCEDE DE PRODUCTION ET LEUR UTILISATION

(30) Priorität: 07.12.2000 DE 10060810
(43) Veröffentlichungstag der Anmeldung: 10.09.2003
(73) Patentinhaber: Sanofi-Aventis Deutschland GmbH, 65929 Frankfurt am Main (DE)
(72) Erfinder: VERTESY, LASZLO, 65817 Eppstein-Vockenhausen (DE); KNAUF, Martin, CH-6037 Root (CH); MARKUS, Astrid, 65835 Liederbach (DE); TOTI, Luigi, 65239 Hochheim am Main (DE); RAYNAL-WETZEL, Mark-Cécile, 75013 Paris (FR); FASSY, Florence, 75019 Paris (FR)
(86) Internationale Anmeldenummer: PCT/EP2001/014013
(87) Internationale Veröffentlichungsnummer: WO 2002/046152

(56) Entgegenhaltungen:
- GB-A- 2 306 476
- US-A- 3 959 468
- J Y LI ET AL: "Cryptocin, a Potent Tetramic Acid Antimycotic from the Endophytic Fungus Cryptosporiopsis cf. quercina" ORGANIC LETTERS, Bd. 2, Nr. 6, 2000, Seiten 767-770, XP002206525 in der Anmeldung erwähnt
- B J L ROYLES : "Naturally Occuring Tetramic Acids: Structure, Isolation, and Synthesis" CHEMICAL REVIEWS, Bd. 95, 1995, Seiten 1981-2001, XP002206526 in der Anmeldung erwähnt
- SINGH S B ET AL: "Equisetin and a Novel Opposite Stereochemical Homolog Phomasetin, Two Fungal Metabolites as Inhibitors of HIV-1 Integrase" TETRAHEDRON LETTERS, ELSEVIER SCIENCE PUBLISHERS, AMSTERDAM, NL, Bd. 39, Nr. 16, 16. April 1998 (1998-04-16), Seiten 2243-2246, XP004111140 ISSN: 0040-4039 in der Anmeldung erwähnt
- T ROSEN ET AL: "Aromatic Dienoyl Tetramic Acids. Novel Antibacterial Agents with Activity against Anaerobes and Staphylococci" JOURNAL OF MEDICINAL CHEMISTRY, Bd. 32, 1989, Seiten 1062-1069, XP002206527

## Beschreibung

Die vorliegende Erfindung betrifft einen neuen Wirkstoff (Coniosetin) vom Tetramsäure-Typ, der von dem Mikroorganismus *Coniochaeta ellipsoidea* Udagawa (DSM 13856) während der Fermentation gebildet wird, von Coniosetin abgeleitete chemische Derivate, ein Verfahren zu deren Herstellung, und die Verwendung der neuen Tetramsäuren als Arzneimittel.

Es ist bereits eine größere Anzahl von Antibiotika mit Tetramsäure-Grundstruktur beschrieben worden. Die Tetramsäure, das 2,4-Pyrrolidindion, ist die Stammverbindung von verschiedenen Naturstoffen, die von einigen Mikroorganismen und marinen Invertebraten gebildet werden. Beschrieben wurde 1994 die
- Harziansäure ( R. Sawa et al. J. Antibiotics, 47, 731-732, 1994), ein sehr wenig wirksames Antibiotikum;

In einen zusammenfassenden Artikel von B.J. L. Royles werden die bis 1994 veröffentlichten natürlichen Tetramsäure-Derivate beschrieben (Chem. Rev. 95, Seite 1981 - 2001, 1995). Seit 1995 wurden weitere natürliche Tetramsäuren beschrieben, aber nur wenige mit antibakteriellen Eigenschaften:
- Reutericyclin (A. Höltzel et al., Angew. Chem. 112, 2886-2888, 2000), eine schwach antibakteriell aktive Verbindung;
- Rubroside A - H, (N. Sata et al., J. Org. Chem. 64, 2331-2339, 1999)
- Aflastatine (M. Ono et al., J. Antibiotics, 51, 1019-1028, 1998)
- F-10778, (A. Tanaka et al., Annu. Rep. Sankyo Res. Lab. 49, 135-141, 1997)
- Ancorinoside A, (S. Ohta et al., J. Org. Chem. 62, 6452-6453, 1997)
- Physarorubinic acid, (A. Nowak et al., Liebigs Ann./Recl. 1997, 1817-1821
- Ascosalipyrrolidinone A, (C. Osterhage et al., J. Org. Chem. 65, 6412-6417, 2000)
- Talaroconvolutine, (S. Suzuki et al., J. Nat. Prod. 63, 768-772, 2000)
- Xanthobaccin A, (Y. Hashidoko et al., Tetrahedron Lett. 40, 2957-2960, 1999)
- Equisetin und Phomasetin (S. S. Singh et al., Tetrahedron Lett. 39, 2243-2246, 1998) sind isomere Inhibitoren der HIV-1 Integrase;
- Cryptocin (J. Y. Li et al., Org. Lett. 2, 767-770, 2000), eine antimycotische Verbindung;
- Vancoresmycin (N. V. S. Ramakrishna et al., Int. Patent Publikation Nr. WO 0028064), ein Antibiotikum.

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antibiotika-gruppen, wie z. B. β-Lactam-Antibiotika, Glycopeptide oder Macrolide widerstands-fähig geworden sind, sonderen gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch, um als Arzneimittel eingesetzt werden zu können.

Es ist überraschend gefunden worden, dass der Stamm *Coniochaeta ellipsoidea* Udagawa (DSM 13856) mindestens ein neues Antibiotikum zu bilden vermag, z.B. das Coniosetin, welches nicht nur antibakteriell sehr wirksam, sondern auch gut verträglich ist.

Gegenstand der Erfindung sind demzufolge die von dem Stamm *Coniochaeta ellipsoidea* Udagawa (DSM 13856) gebildeten Wirkstoffe sowie deren physiologisch verträglichen Salze.

Gegenstand der Erfindung sind dementsprechend Verbindungen der Formel I: 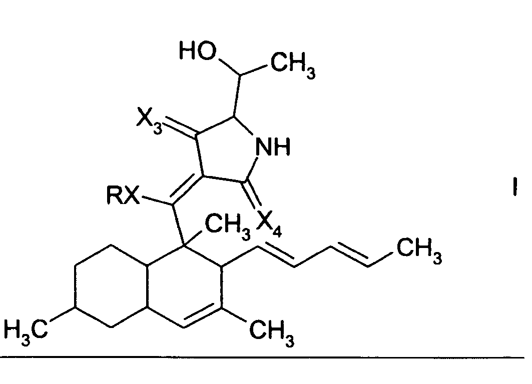 in der bedeuten:
1. H, oder
2. C₁-C₆-Alkyl, C₂-C₆-Alkenyl oder C₂-C₆-Alkinyl, worin Alkyl, Alkenyl und Alkinyl geradkettig oder verzweigt sind und gegebenenfalls ein- oder zweifach substituiert sind durch:
   2.1 -OH,
   2.2 =O,
   2.3 -O-C₁-C₆-Alkyl, worin Alkyl geradkettig oder verzweigt ist,
   2.4 -O-C₂-C₆-Alkenyl, worin Alkenyl geradkettig oder verzweigt ist,
   2.5 -Aryl,
   2.6 -NH-C₁-C₆-Alkyl, worin Alkyl geradkettig oder verzweigt ist,
   2.7 -NH-C₂-C₆-Alkenyl, worin Alkenyl geradkettig oder verzweigt ist,
   2.8 ―NH₂ oder
   2.9 Halogen,
   worin die Substituenten 2.1 bis 2.9 weiter substituiert sein können durch -CN,-Amid oder -Oxim-Funktionen,

X, X₃ und X₄, unabhängig voneinander O, NH, N-C₁-C₆-Alkyl, N-C₂-C₆-Alkenyl, N-C₂-C₆-Alkinyl, N-Acyl, N-Aryl, N-O-R oder S.

Gegenstand der Erfindung sind ferner stereoisomere Formen der Verbindung der Formel I und/oder Gemische diese Formen in jedem Verhältnis, und/oder physiologisch verträgliche Salze der Verbindung der Formel I.

In der Formel I bedeuten
C₁-C₆-Alkyl ein geradkettiges oder verzweigtes Alkyl mit 1 bis 6 C-Atomen, bevorzugt mit 1 bis 4 C-Atomen, wie z.B. Methyl, Ethyl, i-Propyl, tert. Butyl und Hexyl,
C₂-C₆-Alkenyl ein geradkettiges oder verzweigtes Alkenyl mit 2 bis 6 C-Atomen, das einfach, zweifach oder dreifach ungesättigt ist, wie z.B. Allyl, Crotyl, 1-Propenyl, Penta-1,3-dienyl und Pentenyl, und
C₂-C₆- Alkinyl ein geradkettiges oder verzweigtes Alkinyl mit 2 bis 6 C-Atomen, das einfach oder zweifach ungesättigt ist, wie z.B. Propinyl, Butinyl und Pentinyl.

Aryl kann z.B. für Phenyl, Benzyl oder 1- oder 2- Naphthyl, das auch noch substituiert sein kann, beispielsweise durch Halogen, wie Chlor, Brom, Fluor, durch Alkyl mit 1 - 4 C-Atomen, vorzugsweise Methyl-, durch Hydroxy, durch Alkoxy mit 1-4 C Atomen, insbesondere Methoxy und/oder durch Trifluormethyl stehen.

Acyl kann für aliphatische oder aromatische Acyl-Reste stehen. Aliphatisches Acyl hat 1-7, vorzugsweise 1-4 C Atome, wie z.B. Formyl, Acetyl, Propionyl, Butyryl, Hexanoyl, Acryloyl, Crotonoyl, Propioloyl, das noch weiter substituiert sein kann, beispielsweise durch Halogen, wie Chlor, Brom, Fluor, durch Amino, und/oder durch Alkylamino mit 1-4 C Atomen, vorzugsweise Methyl- oder Ethylamino-Gruppen. Aromatisches Acyl kann z.B. Benzoyl oder Naphthoyl sein, das weiter substituiert sein kann, beispielsweise durch Halogen, wie Chlor, Brom, Fluor, durch Alkyl mit 1-4 C-Atomen, vorzugsweise Methyl, durch Hydroxy-, durch Amino-Gruppen, wie z.B. Ethylamino-, oder durch Alkoxy-Gruppen mit 1-7, vorzugsweise 1-4C Atomen, insbesondere Methoxy-.

Die Erfindung betrifft des weiteren eine Verbindung der Formel II 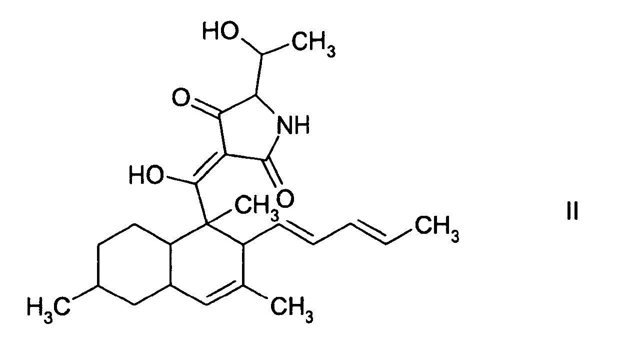 (Coniosetin: Summelformel: C₂₅H₃₅NO₄; MG 413,56) sowie deren physiologisch verträglichen Salze.

Chiralitätszentren in der Verbindungen der Formel I oder II können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Außerdem betrifft die Erfindung eine Verbindung der Summenformel: C₂₅H₃₅NO₄ (Coniosetin) erhältlich durch Fermentierung von *Coniochaeta ellipsoidea* Udagawa (DSM 13856), bis sich die Verbindung Coniosetin in der Kulturbrühe anhäuft und anschließender Isolierung der Verbindung, sowie deren pharmakologisch verträglichen Salzen.

Die Erfindung betrifft weiterhin chemische Derivate, abgeleitet aus einer Verbindung der Summenformel C₂₅H₃₅NO₄ (Coniosetin), erhältlich durch Fermentierung von *Coniochaeta ellipsoidea* Udagawa (DSM 13856), bis sich die Verbindung Coniosetin in der Kulturbrühe anhäuft, Isolierung der Verbindung und anschließender Überführung in chemische Derivate, sowie deren pharmakologisch verträgliche Salze.

Durch die angegebenen Strukturformeln unterscheidet sich das Antibiotikum Coniosetin von literaturbekannten Substanzen. Es sind zwar strukturverwandte Tetramsäure-Derivate beschrieben worden (siehe eine Auswahl an Literaturzitaten oben), sie unterscheiden sich jedoch alle entweder durch die Polarität, durch die chemische Struktur, durch die antimikrobielle Wirksamkeit oder durch weitere physikalische Eigenschaften von den erfindungsgemäßen Verbindungen.

Weiterhin betrifft die Erfindung ein Verfahren zur Herstellung der Verbindung der Formel I, dadurch gekennzeichnet, dass der Mikroorganismus *Coniochaeta ellipsoidea* Udagawa (DSM 13856) in einem wässrigen Nährmedium kultiviert wird, eine Verbindung der Formel I isoliert und gereinigt wird und gegebenenfalls in ihre pharmakologisch verträglichen Salze überführt wird.

Der Stamm *Coniochaeta ellipsoidea* Udagawa (DSM 13856) bildet auf Glukose-, Stärke-, Haferflocken- oder Glycerin-haltigen Nährlösungen das Coniosetin sowie Nebenprodukte.

Ein Isolat von *Coniochaeta ellipsoidea* Udagawa wurde bei der Deutschen Sammlung von Mikroorganismen und Zellkulturen GmbH (DSM), Mascheroder Weg 1 B, 38124 Braunschweig, Deutschland nach den Regeln des Budapester Vertrages am 17.11.2000 unter der Nummer DSM 13856 hinterlegt.

Der Pilz Coniochaeta besitzt ein weisses Substratmycel und sehr wenig Luftmycel. Er bildet in Kultur keine für Coniochaeta charakteristischen Fruchtkörper.

Das besagte Verfahren umfasst die Kultivierung von *Coniochaeta ellipsoidea* Udagawa (DSM 13856), unter aeroben Bedingungen in einem Kohlenstoff- und Stickstoffquelle, anorganischen Salze und gegebenensfalls Spurenelemente enthaltenden Kulturmedium.

Vorzugsweise wird die Kultivierung bei einer Temperatur zwischen 20 und 35°C und bei einem pH zwischen 3 und 10 durchgeführt.

Außerdem betrifft die Erfindung ein Verfahren zur Herstellung einer Verbindung der Formel I, dadurch gekennzeichnet, dass die Verbindung Coniosetin mit einem, Reagenz umgesetzt wird.

Beispielsweise kann eine aktivierte Säure mit Hydroxygruppen oder dem Stickstoff des Coniosetins umgesetzt werden. Aktivierte Säuren sind zum Beispiel Säurechloride oder andere Säure-Derivate, wie sie beispielsweise von Jerry March in der Monographie Advanced Organic Chemistry, John Wiley & Sons, 4^{th} Edition, 1992, beschrieben worden sind. Um Umsetzungen selektiv durchzuführen, kann es vorteilhaft sein, in an sich bekannter Weise vor der Reaktion geeignete Schutzgruppen einzuführen. Die Schutzgruppen werden nach der Reaktion abgespalten und anschließend wird das Reaktionsprodukt gereinigt.

Als bevorzugte Kohlenstoffquellen für die Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt oder Hefextrakt. Als stickstoffhaltige Nährstoffe kommen in Betracht: Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Coniosetine verläuft besonders gut z.B. in einer Nährlösung, die etwa 0,05 bis 5 %, bevorzugt 1 bis 2 % Malzextrakt, etwa 0,05 bis 3 %, bevorzugt 0.05 bis 1 % Hefeextrakt und 0,2 bis 5 %, bevorzugt 0,5 bis 2 % Glucose, 0,5 bis 3 %, bevorzugt 1.5% bis 3 % Haferflocken enthält. Die Angaben in Prozent sind jeweils bezogen auf das Gewicht der gesamten Nährlösung.

In dieser Nährlösung bildet *Coniochaeta ellipsoidea* Udagawa (DSM 13856) ein Gemisch aus Coniosetinen. Je nach Zusammensetzung der Nährlösung kann der mengenmäßige Anteil eines oder mehrerer der erfindungsgemäßen Coniosetine variieren. Außerdem kann durch die Medienzusammensetzung die Synthese einzelner Coniosetine gesteuert werden, so dass ein Coniosetin gar nicht bzw. in einer Menge unterhalb der Nachweisgrenze vom Mikroorganismus hergestellt wird.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 15 bis 35°C, vorzugsweise bei etwa 20 bis 30°C, insbesonders bei 25 bis 30°C durchgeführt werden. Der pH-Bereich sollte zwischen 4 und 10 liegen, vorzugsweise zwischen 6.5 und 7.5. Man kultiviert den Mikroorganismus unter diesen Bedingungen im allgemeinen über einen Zeitraum von 48 bis 720 Stunden, vorzugsweise 72 bis 720 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10 bis 1:100, überimpft werden. Die Vorkultur erhält man z. B., indem man das Mycel in eine Nährlösung überimpft und etwa 20 bis 120 Stunden, bevorzugt 48 bis 72 Stunden, wachsen lässt. Das Mycel kann beispielsweise erhalten werden, indem man den Stamm etwa 1 bis 40 Tage, bevorzugt 21 bis 35 Tage, auf einem festen oder flüssigen Nährboden, beispielsweise Hefe-Malz-Agar, Haferflocken-Agar oder Kartoffeldextrose-Agar, wachsen lässt.

Der Fermentationsverlauf und die Bildung der erfindungsgemäßen Antibiotika kann entsprechend dem Fachmann bekannten Methoden, wie z. B. durch Bestimmung der biologischen Aktivität in Bioassays, oder durch chromatographische Methoden wie Dünnschichtchromatographie (DC) oder Hochleistungsflüssigkeits-Chromatographie (HPLC) verfolgt werden.

Der Pilz *Coniochaeta ellipsoidea* Udagawa (DSM 13856) kann die Verbindung Coniosetin durch eine Oberflächen- oder Standkultur auf festen Nährböden bilden. Feste Nährböden werden durch Zugabe zum Beispiel von Agar oder Gelatine zu wässrigen Nährmedien hergestellt. Es ist aber auch möglich das Coniosetin durch Fermentation des Pilzes *Coniochaeta ellipsoidea* Udagawa im Submersverfahren, d. h. in wässriger Aufschlämmung zu gewinnen. Das Antibiotikum Coniosetin kann sowohl im Mycel als auch im Kulturfiltrat vorkommen, gewöhnlich befindet sich die Hauptmenge in der Zellmasse. Es ist deshalb zweckmäßig, die Fermentationslösung durch Filtration oder Zentrifugation zu trennen. Das Filtrat wird mit einem Adsorptionsharz als feste Phase extrahiert. Das Mycel, aber auch die Oberflächenkultur wird zweckmäßiger Weise mit Methanol oder 2-Propanol extrahiert, es können aber auch andere Lösungsmittel verwendet werden.

Die Extraktionen können in einem weiten pH-Bereich durchgeführt werden, es ist jedoch zweckmäßig, im neutralen oder schwach sauren Milieu, vorzugsweise zwischen pH 3 und pH 7 zu arbeiten. Die Extrakte können z. B. im Vakuum konzentriert und getrocknet werden.

Eine Methode der Isolierung des erfindungsgemäße Antibiotikums ist die Lösungsverteilung in an sich bekannter Weise.

Eine andere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reverse-Phase Träger, z. B. RP₈ und RP₁₈, wie sie z. B. im Rahmen der Hochdruckflüssigkeits-Chromatographie (HPLC) allgemein bekannt geworden sind.

Eine weitere Reinigungsmöglichkeit für das erfindungsgemäße Antibiotikum besteht in der Verwendung von sog. Normal-Phasen-Chromatographie-Trägern, wie z. B. Kieselgel oder Al₂O₃ oder anderen in an sich bekannter Weise.

Ein alternatives Isolierungsverfahren ist die Verwendung von Molekularsieben, wie z. B. Fractogel® TSK HW-40, Sephadex® G-25 und andere, in an sich bekannter Weise. Es ist darüber hinaus auch möglich, aus angereichertem Material das Coniosetin durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.

Coniosetin und, die genannten chemischen Derivate davon können nach dem Fachmann bekannten Methoden in die entsprechenden pharmakologisch verträglichen Salze überführt werden.

Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418, 1985) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, H₂SO₄, Maleinsäure, Fumarsäure in Frage.

Es wurde überraschend gefunden, daß die erfindungsgemäßen Verbindungen starke antibakterielle Wirkungen aufweisen, und sich daher zur Therapie von Erkrankungen eignen, die durch bakterielle Infektionen verursacht werden. Tabelle 1 faßt die Minimalen Hemmkonzentrationen (MHK) von Coniosetin beispielhaft zusammen.

**Tabelle 1: In-Vitro Aktivität (MHK-Werte (µg/ml)) des Coniosetins gegen grampositive Bakterien im Reihenverdünnungstest.**

| | **Resistenz gegen** | **Nach 18 Std** | **24 Std** | **48 Std** |
|---|---|---|---|---|
| *Staphylococcus aureus* | ery S | 0,3 | 0,3 | 0,6 |
| *S. aureus* | oxa S ery R | 0,3 | 0,3 | 0,3 |
| *S. aureus* | oxa R ery R | 0,3 | 0,3 | 0,3 |
| *S. aureus* | oxa S ery R | 0,6 | 0,6 | 0,6 |
| *S. epidermidis* | oxa S ery R | 0,3 | 0,3 | 0,3 |
| *S. aureus* | oxaR eryR tetR | 0,3 | 0,3 | 0,3 |
| *S. aureus* | ofIR oxaR eryR tetR | 0,6 | 0,6 | 0,6 |
| *S. epidermidis* | oxa R ery R | 0,6 | 1,2 | 1,2 |
| *S. pyogenes* | ery S | 0,6 | 1,2 | 1,2 |
| *Enterococcus faecalis* | ery S | 2,5 | 2,5 | 2,5 |
| *Enterococcus faecalis* | ery R | 2,5 | 2,5 | 2,5 |
| *Enterococcus faecium* | teiR vanR eryR tetR | 1,2 | 1,2 | 2,5 |
| *Streptococcus gr.* G | ery S | 1,2 | 1,2 | 1,2 |
| *S. mitis* | ery S | 1,2 | 2,5 | 2,5 |
| *S. pyogenes* | ery R | 1,2 | 1,2 | 1,2 |
| *S. agalactiae* | ery R | 2,5 | 2,5 | 2,5 |
| *Streptococcus gr.G* | ery R | 0,6 | 0,6 | 0,6 |
| *S. sanguis* | ery R | 1,2 | 1,2 | 2,5 |
| *S. mitis* | ery R | 2,5 | 2,5 | 2,5 |
| *S. pneumoniae* | ery S | 0,6 | 0,6 | 0,6 |
| *S. pneumoniae* | ery S pen R | 0,6 | 1,2 | 1,2 |
| *S. pneumoniae* | ery R | 0,6 | 0,6 | 0,6 |
| *S. pneumoniae* | ery R pen R | 0,6 | 0,6 | 0,6 |
| *S. pneumoniae* | ery R pen R | 0,6 | 0,6 | 0,6 |
| *S. pneumoniae* | ery R | 0,6 | 0,6 | 0,6 |
| *S. pneumoniae* | ery R | 0,6 | 0,6 | 1,2 |
| *Escherichia coli* | | >40 | | |

| | | | | |
|---|---|---|---|---|
| S = sensitiv, R = resistent, ery = Erythromycin, ofl= Ofloxacin, oxa = Oxacillin, pen = Penicillin, tei = teicoplanin, van = Vancomycin, tet = Tetracyclin. | | | | |

Es ist besonders bemerkenswert, dass die erfindungsgemäße Verbindung keinerlei Kreuzresistenz mit herkömmlichen Antibiotika, wie zum Beispiel den β-Lactamen (Penicilline, Cephalosporine), Aminoglycosiden (Streptomycin), Makroliden (Erythromycin), Chinolonen (Ciprofloxacin), Sulfonamiden oder Glycopeptiden (Vancomycin) und anderen aufweist.

Hervorzuheben ist darüber hinaus eine - wenngleich schwächere - Hemmwirkung auf Hefen, wie zum Beispiel *Candida albicans* und gegen Pilze, wie *Aspergillus niger,* die hartnäckige, ja sogar lebensbedrohende Infektionskrankheiten verursachen können (Beispiel 8). Zur Therapie solcher Erkrankungen eignet sich Coniosetin daher ebenfalls.

Coniosetin ist in der wirksamen Konzentration und bei höheren Konzentrationen gut verträglich.

Die vorliegende Erfindung betrifft demzufolge auch die Anwendung der erfindungsgemäßen Verbindungen als Arzneimittel, sowie die Verwendung der betreffenden Verbindungen zur Herstellung von Arzneimitteln zur Behandlung und/oder zur Prophylaxe von bakteriellen Infektionen und Mycosen.

Des weiteren betrifft die vorliegende Erfindung Arzneimittel mit einem Gehalt an der erfindungsgemäßen Verbindung.

Das besagte Arzneimittel wird durch Mischung von mindestens einer Verbindung der Formel I mit einem physiologischen Hilfs- und/oder Trägerstoff hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvern Tabletten, Kapseln (einschließlich Mikrokapseln), Salben (Cremes oder Gel), oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 -1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Die folgenden Beispiele sollen der näheren Erläuterung der Erfindung dienen, ohne die Breite der Erfindung in irgendeiner Weise begrenzen zu wollen.

### Beispiel 1 Herstellung einer Glycerinkultur von Coniochaeta ellipsoidea (DSM 13856)

30 ml Nährlösung (Malzextrakt 2,0%, Hefeextrakt 0,2 %, Glucose 1,0 % (NH₄)₂HPO₄ 0,05 %, pH 6,0) in einem sterilen 100 ml Erlenmeyerkolben werden mit dem Stamm *Coniochaeta ellipsoidea* Udagawa (DSM 13856), beimpft und 6 Tage bei 25°C und 140 UpM auf einer rotierenden Schüttelmaschine inkubiert. 1,5 ml dieser Kultur werden anschließend mit 2,5 ml 80 %igem Glycerin verdünnt und bei -135°C gelagert.

### Beispiel 2 Herstellung einer Vorkultur im Erlenmeyerkolben von Coniochaeta ellipsoidea Udagawa (DSM 13856).

100 ml Nährlösung (Malzextrakt 2,0%, Hefeextract 0,2 %, Glucose 1,0 % (NH₄)₂HPO₄ 0,05 %, pH 6,0) in einem sterilen 300 ml Erlenmeyerkolben werden mit dem Stamm *Coniochaeta ellipsoidea* Udagawa (DSM 13856), beimpft und 4 Tage bei 25° C und 140 UpM auf einer rotierenden Schüttelmaschine inkubiert. 2 ml dieser Vorkultur werden anschließend für die Herstellung der Hauptkulturen beimpft.

### Beispiel 3 Herstellung einer Hauptkultur von Coniochaeta ellipsoidea Udagawa (DSM 13856) auf Festmedium Platten.

30 sterile 25 x 25 cm Platten werden mit 200 ml einer Nährlösung enthaltend 20 g/l Malzextrakt, 20 g/l Haferflocken, 2% Agar und eines pH Wertes 7.0 gegossen. Diese Platten werden mit 2 ml einer Vorkultur beimpft und bei 25° C inkubiert. Die maximale Produktion einer oder mehrerer Verbindungen der erfindungsgemäßen Coniosetins ist nach ca. 676 Stunden erreicht.

### Beispiel 4: Isolierung des Antibiotikums Coniosetin.

30 Agarplatten, Größe je 25 x 25 cm, gewonnen nach Beispiel 3, werden gefriergetrocknet und mit 2.5 Liter Methanol extrahiert. Die klare flüssige Phase wird im Vakuum auf 100 mL eingeengt, mit Wasser verdünnt und auf eine 580 mL fassende, mit dem Adsorptionsharz MCI Gel® CHP20P gefüllte Säule aufgetragen. Säulenmaße: Breite x Höhe: 5 cm x 30 cm. Eluiert wird mit einem Lösungsmittel-Gradienten von 5 % Acetonitril in Wasser bis 90 % Acetonitril und der Säulenausfluß (40 mL/Minute) in Fraktionen je 120 mL aufgefangen. Die Coniosetin-haltigen Fraktionen, die durch HPLC-Analysen überprüft werden, werden gesammelt und im Vakuum konzentriert sowie gefriergetrocknet (0.3 g).

### Beispiel 5: Hochdruckflüssigkeitschromatographie (HPLC) des Coniosetins.

| | |
|---|---|
| Säule: | Superspher 100 RP-18e®, 250-4, mit Vorsäule, |
| Mobile Phase: | 75 % Acetonitril in 0.1 % Phosphorsäure, |
| Flußgeschwindigkeit: | 1 mL pro Minute, |

Detektion durch UV-Absorption bei 210 nm.

Die Retentionszeit von Coniosetin beträgt 13.6 Minuten.

### Beispiel 6: Endreinigung des Coniosetins.

Das angereicherte Antibiotikum Coniosetin (0.3 g), gewonnen nach Beispiel 4, wird auf einer LiChrospher® 100 RP-18e-HPLC-Säule (Breite x Höhe = 2.5 cm x 25 cm) im Gradientenverfahren mit 75 % bis 100 % Acetonitril in 0,05 % Essigsäure aufge-trennt. Fluß: 30 mL/Min. Fraktionsgröße: 60 mL. Die durch analytische HPLC (siehe Beispiel 5) untersuchten Fraktionen werden entsprechend ihrem Coniosetin-Gehalt zusammengefaßt, im Vakuum eingeengt und gefriergetrocknet. Sie ergeben 170 mg Coniosetin in 98 %iger Reinheit.

### Beispiel 7: Massenspektrometrische Charakterisierung des Coniosetins.

Bestimmung des Molpeaks:
Dem gesuchten Molekül wird die Masse 413 zugeordnet aufgrund folgender Befunde: ESI⁺-Spektrum sowie FAB⁺-Spektren zeigen Peaks bei 414 amu (M+H)⁺. ESI⁻ -Spektrum zeigt unter anderem einen Peak bei 412 amu (M-H)⁻.
Hochauflösung des Quasi-Molekülions:
   Unter FAB-Bedingungen mit einer Nitrobenzylalkohol-Matrix wird u.a. ein Peak bei 414.2645 amu beobachtet. Die bei der Messung vorliegenden Massen-genauigkeit beträgt ca. 5 ppm. Der Meßwert stimmt gut mit dem für C₂₅H₃₆NO₄ = 414.2644 amu berechneten Elementarzusammensetzung überein. Bei dieser Elementarzusammensetzung liegen 9 Doppelbindungsäquivalente vor.

MS/MS-Experimente mit einem lon-Trap-Massenspektrometer führen zu folgenden Fragmentierungen im ESI⁺-Modus:
414 amu zu 396 amu (-H₂O), 386 amu (-CO), 370 amu (-C₂H₄O), 346 amu (-C₅H₈), 278 amu, 271 amu, 245 amu, 215 amu, 196 amu und weitere Fragmente geringer Intensität.
396 amu zu 378 amu (-H₂O), 352 amu (-C₂H₄O), 253 amu, 241 amu, 227 amu 386 amu zu 368 amu (-H₂O), 243 amu .

Die physikalisch-chemischen sowie spektroskopischen Eigenschaften des erfindungsgemäßen Antibiotikums lassen sich wie folgt zusammenfassen:

### Coniosetin:

Aussehen:
Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
Summenformel: C₂₅H₃₅NO₄
Molekulargewicht: 413,56
¹H- und ¹³C-NMR: siehe Tabelle 2 und 3
UV-Maxima: 233 nm, 288 nm

**Tabelle 2: ¹H- und ¹³C- chemische Verschiebungen von Coniosetin in DMSO-d₆ und Methanol-d₄ bei 300K.**

| | **DMSO-d**_{**6**} | | **Methanol-d**_{**4**} | |
|---|---|---|---|---|
| **Position** | ^{**13**}**C** | ^{**1**}**H** | ^{**13**}**C** | ^{**1**}**H** |
| | **δ (ppm)** | **δ (ppm)** | **δ (ppm)** | **δ (ppm)** |
| 1 | 48.94 | - | 51.18 | - |
| 1-Me | 13.34 | 1.33 s | 14.31 | 1.42 s, br |
| 2 | 48.45 | 3.19 | 50.59 | 3.28 br |
| 3 | 130.99 | - | 133.15 | - |
| 3-Me | 22.04 | 1.53 t | 22.67 | 1.58 t |
| 4 | 125.88 | 5.19 s, br | 127.31 | 5.20s |
| 5 | 38.61 | 1.80 | 40.68 | 1.86 m |
| 6 | 42.06 | 1.78,0.82 | 44.10 | 1.83 d, br, 0.87 m |
| 7 | 32.90 | 1.49 | 35.00 | 1.52 m, br |
| 7-Me | 22.40 | 0.89 d | 23.07 | 0.94 d |
| 8 | 35.44 | 1.72, 1.01 | 37.20 | 1.77 d, br, 1.10 m |
| 9 | 27.59 | 1.94 d, 1.00 | 29.47 | 2.01 d, br, 1.06 m |
| 10 | 39.28 | 1.57 | 41.39 | 1.66 m |
| 11 | 130.42 | 5.18 m | 132.05 | 5.19 |
| 12 | 131.96 | 5.72t | 134.03 | 5.78t |
| 13 | 131.31 | 5.91t | 132.71 | 5.90t |
| 13a | 127.83 | 5.52 m | 129.12 | 5.51 m |
| 13b | 17.73 | 1.65 d | 18.23 | 1.67 d |
| 14 | 198.23 | - | 201.30 | - |
| 14-OH | - | 17.49 s, br | - | - |
| 15 | 99.46 | - | 101.50 | - |
| 16 | 179.52 | - | 181.53 | - |
| 17 | - | 9.22 s, br | - | - |
| 18 | 66.57 | 3.62 | 68.19 | 3.62 br |
| 19 | 191.09 | - | 193.51 | - |
| 20 | 65.66 | 3.91 | 68.11 | 4.06 br |
| 20-OH | - | 4.76 d | - | - |
| 21 | 20.67 | 1.17 d | 20.65 | 1.29 d, br |

**Tabelle 3: ¹H- und ¹³C- chemische Verschiebungen von Conisetin in CDCl₃ bei 300K.**

| **Position** | ^{**13**}**C** | ^{**1**}**H** |
|---|---|---|
| | **δ (ppm)** | **δ(ppm)** |
| 1 | 49.84 | - |
| 1-Me | 13.76 | 1.44 |
| 2 | 49.25 | 3.22 |
| 3 | 131.50 | - |
| 3-Me | 22.23 | 1.61 |
| 4 | 126.01 | 5.20 |
| 5 | 39.13 | 1.85 |
| 6 | 42.54 | 1.82, 0.90 |
| 7 | 33.54 | 1.54 |
| 7-Me | 22.46 | 0.94 |
| 8 | 35.78 | 1.79, 1.13 |
| 9 | 28.30 | 1.99, 1.08 |
| 10 | 39.73 | 1.68 |
| 11 | 130.19 | 5.23 |
| 12 | 132.62 | 5.83 |
| 13 | 131.39 | 5.89 |
| 13a | 128.19 | 5.52 |
| 13b | 18.02 | 1.70 |
| 14 | 200.23 | - |
| 14-OH | - | - |
| 15 | 100.28 | - |
| 16 | 179.33 | - |
| 17 | - | 6.19 |
| 18 | 65.43 | 3.71 |
| 19 | 190.95 | - |
| 20 | 67.80 | 4.05 |
| 20-OH | - | - |
| 21 | 19.56 | 1.34 |

### Beispiel 8: Hemmwirkung des Coniosetins im Agar-Diffusionstest.

Es werden Agarplatten zubereitet mit 2 mL *Staphylococcus aureus*-Einsaat in 200 mL Agarlösung, 2 mL *Escherichia coli*-Einsaat in 200 mL Agar; 2 mL *Candida albicans-*Einsaat in 200 mL Agarlösung; 1 mL *Aspergillus niger*-Einsaat in 200 mL AgarLösung und 3 mL *Streptomyces murinus*-Einsaat in 200 mL Agarlösung. Das Coniosetin wird in einer 10 mM Lösung auf Blättchen mit einem Durchmesser von 6 mm aufgetragen und auf die Agarplatte gelegt. Die beimpften *Staphylococcus-, E. coli-* und *Candida-*Platten werden 16 Stunden bei 37°C, die A. *niger-*Platte 40 Stunden bei 28°C und die *Streptomyces*-Platte 16 Stundfen bei 28°C inkubiert. Dann werden Hemmhöfe mit folgenden Durchmessern (mm) beobachtet.

| Menge | *S*. *aureus* | *E. coli* | *C. albicans* | *A. niger* | *S. murinus* |
|---|---|---|---|---|---|
| 10 µL | 13 | 0 | 9 | 0 | 0 |
| 20 µL | 14.5 | 0 | 10 | 0 | 9 |
| 40 µL | 15.5 | 0 | 10.5 | 8 | 10 |

## Patentansprüche

1. Verbindung der allgemeinen Formel I 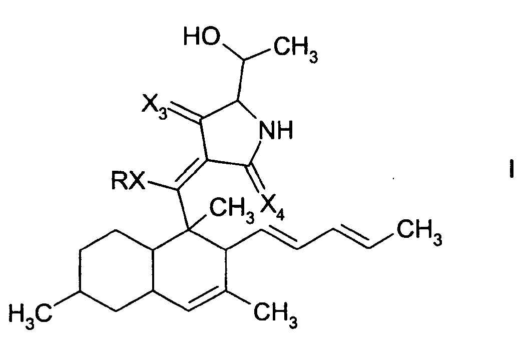 wobei:
R
1. H, oder
2. C₁-C₆-Alkyl, C₂-C₆-Alkenyl oder C₂-C₆-Alkinyl, worin Alkyl, Alkenyl und Alkinyl geradkettig oder verzweigt sind und gegebenenfalls ein- oder zweifach substituiert sind durch:
2.1 -OH,
2.2 =O,
2.3 -O-C₁-C₆-Alkyl, worin Alkyl geradkettig oder verzweigt ist,
2.4 -O-C₂-C₆-Alkenyl, worin Alkenyl geradkettig oder verzweigt ist,
2.5 -Aryl,
2.6 -NH-C₁-C₆-Alkyl, worin Alkyl geradkettig oder verzweigt ist,
2.7 -NH-C₂-C₆-Alkenyl, worin Alkenyl geradkettig oder verzweigt ist,
2.8 -NH₂ oder
2.9 Halogen,
worin die Substituenten 2.1 bis 2.9 weiter substituiert sein können durch -CN, -Amid oder -Oxim-Funktionen,
X, X₃ und X₄, unabhängig voneinander O, NH, N-C₁-C₆-Alkyl, N-C₂-C₆-Alkenyl, N-C₂-C₆-Alkinyl, worin Alkyl, Alkenyl und Alkinyl geradkettig oder verzweigt sind, N-Acyl, N-Aryl, N-O-R oder S,
bedeuten,
und/oder eine stereoisomere Form der Verbindung der Formel Iund/oder Gemische dieser Form in jedem Verhältnis und/oder ein physiologisch verträgliche Salz der Verbindung der Formel I.

2. Verbindung der Formel II gemäß Anspruch 1 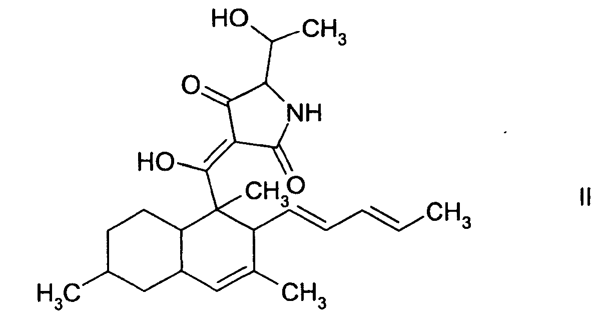 und/oder eine stereoisomere Form der Verbindung der Formel II und/oder Gemische dieser Form in jedem Verhältnis und/oder ein physiologisch verträgliche Salz der Verbindung der Formel II.

3. Verbindung der Summenformel: C₂₅H₃₅NO₄ (Coniosetin) erhältlich durch Fermentierung von *Coniochaeta ellipsoidea* Udagawa (DSM 13856), bis sich die Verbindung Coniosetin in der Kulturbrühe anhäuft und anschließender Isolierung der Verbindung, sowie deren pharmakologisch verträgliche Salze.

4. Chemische Derivate, abgeleitet aus einer Verbindung der Summenformel C₂₅H₃₅NO₄ (Coniosetin), erhältlich durch Fermentierung von *Coniochaeta ellipsoidea* Udagawa (DSM 13856), bis sich die Verbindung Coniosetin in der Kulturbrühe anhäuft, Isolierung der Verbindung und anschließender Überführung in chemische Derivate, sowie deren pharmakologisch verträglichen Salze.

5. Verfahren zur Herstellung einer Verbindung gemäß Anspruch 1, **dadurch gekennzeichnet, dass** Coniosetin mit einem Carbonsäure-Derivat umgesetzt wird und gegebenenfalls in ein pharmakologisch verträgliches Salz überführt wird.

6. Verfahren zur Herstellung einer Verbindung gemäß einem oder mehreren der Ansprüche 1 bis 3, **dadurch gekennzeichnet, dass** der Mikroorganismus *Coniochaeta ellipsoidea* Udagawa (DSM 13856) in einer Kultur fermentiert wird, eine Verbindung der Formel I isoliert wird und gegebenenfalls in ihre pharmakologisch verträglichen Salze überführt wird.

7. Verfahren gemäß Anspruch 6, **dadurch gekennzeichnet, dass** man *Coniochaeta ellipsoidea* Udagawa (DSM 13856) in eine Kohlenstoff- und Stickstoffquelle sowie die üblichen anorganischen Salze und Spurenelemente enthaltenden Kulturmedien unter aeroben Bedingungen fermentiert.

8. Verfahren gemäß einem oder mehreren der Ansprüche 6 oder 7, **dadurch gekennzeichnet, dass** die Fermentierung in einem Nährmedium, das als Kohlenstoffquelle 0,5 bis 5 % Malzextrakt und 0,5 bis 5 % Haferflocken enthält, durchgeführt wird.

9. Verfahren gemäß einem oder mehreren der Ansprüche 6 bis 8, **dadurch gekennzeichnet, dass** die Fermentation unter aeroben Bedingungen bei einer Temperatur zwischen 20 und 35°C und bei einem pH zwischen 4 und 10 durchgeführt wird.

10. Verbindung gemäß einem oder mehreren der Ansprüche 1 bis 4 zur Anwendung als Arzneimittel.

11. Verwendung einer Verbindung gemäß einem oder mehreren der Ansprüche 1 bis 4 zur Herstellung eines Arzneimittels zur Behandlung und Prophylaxe von bakteriellen Infektionskrankheiten.

12. Verwendung einer Verbindung gemäß einem oder mehreren der Ansprüche 1 bis 4 zur Herstellung eines Arzneimittels zur Behandlung und Prophylaxe von Mycosen.

13. Arzneimittel mit einem Gehalt an mindestens einer Verbindung gemäß einem oder mehreren der Ansprüche 1 bis 4 und einer oder mehrere physiologisch annehmbare Träger sowie gegebenenfalls geeignete Hilfsstoffe.

14. Verfahren zur Herstellung eines Arzneimittels gemäß Anspruch 13, **dadurch gekennzeichnet, dass** man mindestens eine Verbindung gemäß einem oder mehreren der Ansprüche 1 bis 4 mit einem physiologischen Hilfs- und/oder Trägerstoff in eine geeignete Darreichungsform bringt.

15. Verwendung von *Coniochaeta ellipsoidea* Udagawa (DSM 13856) zur Gewinnung von Antibiotika.

16. *Coniochaeta ellipsoidea* Udagawa (DSM 13856).

## Claims

1. A compound of the formula I 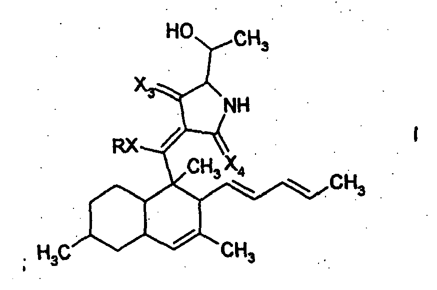 in which:
R is
1. H, or
2. C₁-C₆-alkyl, C₂-C₆-alkenyl or C₂-C₆-alkynyl, in which alkyl, alkenyl and alkynyl are straight-chain or branched and are optionally substituted once or twice by:
2.1 -OH,
2.2 =O,
2.3 -O-C₁-C₆-alkyl, in which alkyl is straight-chain or branched,
2.4 -O-C₂-C₆-alkenyl, in which alkenyl is straight-chain or branched,
2.5 -aryl,
2.6 -NH-C₁-C₆-alkyl, in which alkyl is straight-chain or branched,
2.7 -NH-C₂-C₆-alkenyl, in which alkenyl is straight-chain or branched,
2.8 -NH₂ or
2.9 halogen,
in which substituents 2.1 to 2.9 may be further substituted by -CN, amide or oxime functions,
X, X₃ and X₄ are, independently of one another, O, NH, N-C₁-C₆-alkyl, N-C₂-C₆-alkenyl, N-C₂-C₆-alkynyl, in which alkyl, alkenyl and alkynyl are straight-chain or branched, N-acyl, N-aryl, N-O-R or S,
and/or a stereoisomeric form of the compound of the formula I and/or mixtures of this form in any ratio and/or a physiologically tolerated salt of the compound of the formula I.

2. A compound of the formula II as claimed in claim 1 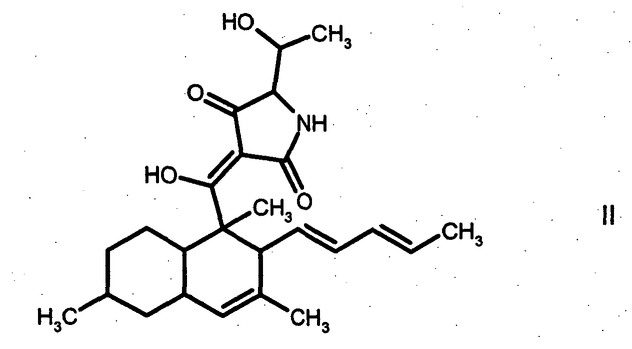 and/or a stereoisomeric form of the compound of the formula II and/or mixtures of this form in any ratio and/or a physiologically tolerated salt of the compound of the formula II.

3. A compound of the molecular formula: C₂₅H₃₅NO₄ (coniosetin) obtainable by fermentation of *Coniochaeta ellipsoidea* Udagawa (DSM 13856) until the compound coniosetin accumulates in the culture broth and subsequent isolation of the compound, and the pharmacologically acceptable salts thereof.

4. A chemical derivative derived from a compound of the molecular formula C₂₅H₃₅NO₄ (coniosetin), obtainable by fermentation of *Coniochaeta ellipsoidea.* Udagawa (DSM 13856) until the compound coniosetin accumulates in the culture broth, isolation of the compound and subsequent conversion into a chemical derivative, and the pharmacologically acceptable salts thereof.

5. A process for preparing a compound as claimed in claim 1, which comprises reacting coniosetin with a carboxylic acid derivative and, where appropriate, converting into a pharmacologically acceptable salt.

6. A process for preparing a compound as claimed in one or more of claims 1 to 3, which comprises fermenting the microorganism *Coniochaeta ellipsoidea* Udagawa (DSM 13856) in a culture, isolating a compound of the formula I and, where appropriate, converting into its pharmacologically acceptable salts.

7. The process as claimed in claim 6, wherein *Coniochaeta ellipsoidea* Udagawa (DSM 13856) is fermented in culture media containing a carbon and nitrogen source and the customary inorganic salts and trace elements under aerobic conditions.

8. The process as claimed in one or more of claims 6 or 7, wherein the fermentation is carried out in a nutrient medium which contains 0.5 to 5% malt extract and 0.5 to 5% oatmeal as carbon source.

9. The process as claimed in one or more of claims 6 to 8, wherein the fermentation is carried out under aerobic conditions at a temperature between 20 and 35°C and at a pH between 4 and 10.

10. A compound as claimed in one or more of claims 1 to 4 for use as pharmaceutical.

11. The use of a compound as claimed in one or more of claims 1 to 4 for producing a pharmaceutical for the treatment and prophylaxis of bacterial infectious diseases.

12. The use of a compound as claimed in one or more of claims 1 to 4 for producing a pharmaceutical for the treatment and prophylaxis of mycoses.

13. A pharmaceutical with a content of at least one compound as claimed in one or more of claims 1 to 4. and one or more physiologically acceptable carriers and, where appropriate, suitable excipients.

14. A process for producing a pharmaceutical as claimed in claim 13, which comprises converting at least one compound as claimed in one or more of claims 1 to 4 into a suitable dosage form with a physiological excipient and/or carrier.

15. The use of *Coniochaeta ellipsoidea* Udagawa (DSM 13856) for obtaining antibiotics.

16. *Coniochaeta ellipsoidea* Udagawa (DSM 13856).

## Revendications

1. Composé de formule générale I 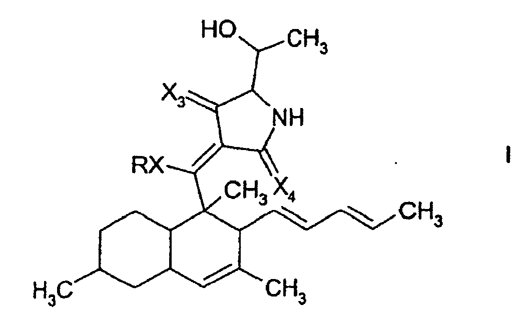 dans laquelle :
R représente
1. H ou
2. un groupe alkyle en C₁-C₆, alcényle en C₂-C₆ ou alcynyle en C₂-C₆, les groupes alkyle, alcényle et alcynyle étant à chaîne droite ou ramifiée et étant éventuellement une ou deux fois substitués par :
2.1 -OH,
2.2 =O,
2.3 -O-alkyle(C₁-C₆), le fragment alkyle étant à chaîne droite ou ramifiée,
2.4 -O-alcényle(C₂-C₆), le fragment alcényle étant à chaîne droite ou ramifiée,
2.5 -aryle,
2.6 -NH-alkyle(C₁-C₆), le fragment alkyle étant à chaîne droite ou ramifiée,
2.7. -NH-alcényle (C₂-C₆), le fragment alcényle étant à chaîne droite ou ramifiée,
2.8 -NH₂ ou
2.9 halogène,
les substituants 2.1 à 2.9 pouvant être encore substitués par des fonctions -CN, -amide ou -oxime,
X, X₃ et X₄ représentent, chacun indépendamment, O, NH, N-alkyle(C₁-C₆), N-alcényle(C₂-C₆), N-alcynyle(C₂-C₆), les fragments alkyle, alcényle et alcynyle étant à chaîne droite ou ramifiée, N-acyle, N-aryle, N-O-R ou S,
et/ou forme stéréo-isomère du composé de formule I et/ou mélanges de telles formes en tout rapport et/ou sel physiologiquement acceptable du composé de formule I.

2. Composé de formule II selon la revendication 1 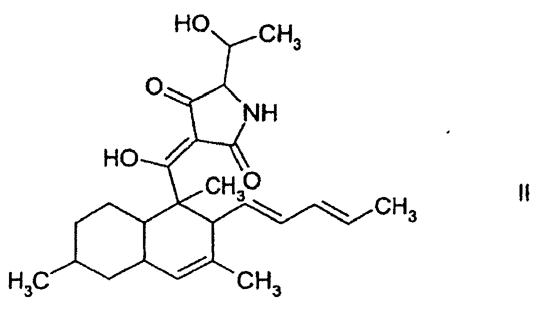 et/ou forme stéréo-isomère du composé de formule II et/ou mélanges de telles formes en tout rapport et/ou sel physiologiquement acceptable du composé de formule II.

3. Composé de formule brute : C₂₅H₃₅NO₄ (coniosétine), pouvant être obtenu par culture par fermentation de *Coniochaeta ellipsoidea* Udagawa (DSM 13856), jusqu'à ce que le composé coniosétine s'accumule dans le bouillon de culture et isolement subséquent du composé ainsi que de ses sels pharmacologiquement acceptables.

4. Dérivés chimiques issus d'un composé de formule brute C₂₅H₃₅NO₄ (coniosétine), pouvant être obtenu par culture par fermentation de *Coniochaeta ellipsoidea* Udagawa (DSM 13856), jusqu'à ce que le composé coniosétine s'accumule dans le bouillon de culture, isolement du composé et transformation subséquente en dérivés chimiques, et sels pharmacologiquement acceptables de tels dérivés.

5. Procédé pour la préparation d'un composé selon la revendication 1, **caractérisé en ce qu'**on fait réagir de la coniosétine avec un dérivé d'acide carboxylique et éventuellement on la convertit en un sel pharmacologiquement acceptable.

6. Procédé pour la préparation d'un composé selon une ou plusieurs des revendications 1 à 3, **caractérisé en ce qu'**on cultive par fermentation le micro-organisme *Coniochaeta ellipsoidea* Udagawa (DSM 13856), on isole un composé de formule I et éventuellement on le convertit en ses sels pharmacologiquement acceptables.

7. Procédé selon la revendication 6, **caractérisé en ce qu'**on cultive par fermentation, dans des conditions aérobies, *Coniochaeta ellipsoidea* Udagawa (DSM 13856) dans des milieux de culture contenant une source de carbone et d'azote ainsi que les sels minéraux et oligoéléments usuels.

8. Procédé selon une ou plusieurs des revendications 6 et 7, **caractérisé en ce que** la fermentation est effectuée dans un milieu nutritif qui contient en tant que source de carbone 0,5 à 5 % d'extrait de malt et 0,5 à 5 % de flocons d'avoine.

9. Procédé selon une ou plusieurs des revendications 6 à 8, **caractérisé en ce que** la fermentation est effectuée dans des conditions aérobies, à une température comprise entre 20 et 35°C et à un pH compris entre 4 et 10.

10. Composé selon une ou plusieurs des revendications 1 à 4, pour utilisation en tant que médicament.

11. Utilisation d'un composé selon une ou plusieurs des revendications 1 à 4, pour la fabrication d'un médicament destiné au traitement et à la prophylaxie de maladies dues à des infections bactériennes.

12. Utilisation d'un composé selon une ou plusieurs des revendications 1 à 4, pour la fabrication d'un médicament destiné au traitement et à la prophylaxie de mycoses.

13. Médicament contenant au moins un composé selon une ou plusieurs des revendications 1 à 4 et un ou plusieurs véhicules physiologiquement acceptables ainsi qu'éventuellement des adjuvants convenables.

14. Procédé pour la fabrication d'un médicament selon la revendication 13, **caractérisé en ce qu'**ont met sous une forme d'administration appropriée au moins un composé selon une ou plusieurs des revendications 1 à 4 avec un adjuvant et/ou véhicule physiologique.

15. Utilisation de *Coniochaeta ellipsoidea* Udagawa (DSM 13856), pour l'obtention d'antibiotiques.

16. *Coniochaeta ellipsoidea* Udagawa (DSM 13856).
